# EUROPEAN PATENT SPECIFICATION

(11) **EP 1 429 733 B1**
(45) Date of publication and mention of the grant of the patent: **10.12.2008**
(21) Application number: 02749087.9
(22) Date of filing: 31.07.2002
(51) Int. Cl.: A61K 9/16

(54) **PREPARATION OF MICROPARTICLES**
HERSTELLUNG VON MIKROPARTIKELN
PRODUCTION DE MICROPARTICULES

(30) Priority: 17.08.2001 GB 0120123
(43) Date of publication of application: 23.06.2004
(73) Proprietor: Upperton Limited, Nottingham NG2 5NA (GB)
(72) Inventor: JOHNSON, Richard Alan, Nottingham NG2 5NA (GB)
(74) Representative: Jones, Stephen Anthony
(86) International application number: PCT/GB2002/003491
(87) International publication number: WO 2003/015756

(56) References cited:
- EP-A- 0 611 567
- WO-A-00/10541
- WO-A-01/13891
- WO-A-01/13892
- WO-A-98/31346
- WO-A-99/29320
- US-A- 4 826 689

## Description

This invention relates to the preparation of microparticles, and in particular to the preparation of proteinaceous material in the form of fine particles.

The preparation of microparticles of proteinaceous materials such as albumin is well-documented. A technique that has been used is spray-drying, in which a solution of the proteinaceous material in a solvent, most commonly water, is sprayed into a heated gas-filled chamber such that the solvent evaporates to form microparticles which are then separated from the gas.

There is continued interest in developing new and improved techniques for making microparticles containing both drugs and imaging agents, and this involves numerous challenges. For instance, there is a need to control the particle size distribution closely. For products intended for intravenous administration, it is desirable to make particles less than 6µm in size. Similarly, for products intended for delivery by inhalation to the lung it is usually beneficial to produce microparticles with an aerodynamic size suitable for penetration deep into the lung. Particles for nasal administration may be rather larger, eg with a particle size of several tens of µm.

Similarly, there may be a need to encapsulate or otherwise incorporate therapeutic agents (drugs) or contrast agents. By encapsulating drugs and contrast agents it is hoped that they will remain in the circulation for longer, enhancing performance and possibly reducing toxicity.

WO 01/13892 discloses the formation of particles, having a tap density less than about 0.4gcm⁻³, by spray-drying from a colloidal solution including a carboxylic acid or salt thereof, a phosopholipid, a divalent salt and a solvent.

EP 0611567A1 refers to an ultrafine powder for inhalation comprising at least one cellulose lower alkyl ether selected from hydroxylpropyl cellulose and hydroxylpropyl methyl cellulose, and a medicament, and a method for the production thereof.

WO 99/29320 relates to oral administration forms for acid-labile compounds.

As well as the spray-drying techniques noted above, a number of other methods have been used to make microparticles smaller than 10µm, for both imaging and therapeutic purposes. These methods include emulsification, precipitation and milling. However, all these existing methods still have a number of disadvantages:
(i) Presence of significant amounts of particles over 6µm in size. This is important since if administered intravenously these larger particles will block capillaries etc, and if administered to the lung will fail to penetrate deep into the airways.
(ii) A requirement for the incorporation of surfactants to avoid agglomeration and to maintain a relatively homogeneous suspension - the presence of such additional ingredients, which are of no therapeutic or functional benefit, and which could be harmful, is generally undesirable.
(iii) The products obtained in some emulsion-based processes may be relatively hydrophobic, and consequently difficult to disperse.

There has now been devised an improvement to known spray-drying processes for the preparation of microparticles, that overcomes or substantially mitigates the above-mentioned and/or other disadvantages of the prior art.

According to the invention, there is provided a method for producing microparticles of a particle-forming material, which method comprises the steps of
a) forming a suspension of the particle-forming material; and
b) spray-drying said suspension;
characterised in that step a) is carried out by first dissolving the particle-forming material in a solvent, and then adding to the solution so formed a non-solvent for the particle-forming material, so as to bring about precipitation of the particle-forming material.

By a "non-solvent" is meant a liquid in which the solubility of the particle-forming material is substantially less than the solubility of the particle-forming material in the solvent, but which is miscible with the solvent.

The non-solvent is preferably added in excess, ie the volume of non-solvent added to the solvent is preferably greater than the volume of the solution of the particle-forming material in the solvent. In other words, the solvent / non-solvent mixture that is spray-dried in step b) most preferably comprises in excess of 50% v/v of non-solvent, more preferably in excess of 60% v/v, and possibly in excess of 70% v/v.

Most commonly, the solvent is water. The preferred non-solvent is ethanol. In general, however, any suitable combination of solvent and non-solvent may be used, provided that the addition of non-solvent has the desired effect of causing precipitation of the particle-forming material and that the solvent and the non-solvent are miscible in the proportions used.

Although the solvent is most commonly water, it may alternatively be, for example, an organic solvent. In such a case, the non-solvent may be water, and the use of the non-solvent may then be beneficial in reducing risks associated with the subsequent spray-drying of the suspension containing the possibly flammable organic solvent.

Where the particle-forming material is, as is preferred, a proteinaceous material, the precipitation by addition of the non-solvent is preferably carried out at a pH which is removed from the isoelectric point, so as to prevent or minimise agglomeration of the suspended particles and to produce hydrophilic particles that are readily susceptible to dispersion after spray-drying. In this way, the use of additional surfactants to achieve the same objectives may be avoided. The same may be true in the case of non-proteinaceous particle-forming materials, if those materials too exhibit an isoelectric point.

Step b), ie spray-drying of the suspension formed in step a), may be carried out in a generally conventional manner, using equipment of a generally conventional nature. In outline, the spray-drying process involves spraying the suspension into a chamber containing a heated gas, most commonly air. This causes the solvent/non-solvent mixture to evaporate and produces solid microparticles. The gas is drawn from the chamber, and the microparticles entrained in the gas are separated from the gas, eg by means of cyclonic separator or some form of filter arrangement. The microparticles are then collected in a suitable receptacle.

The properties of the microparticles obtained by the spray-drying process are dependent on a number of factors. These include the flow rate of gas through the spray-drying apparatus, the concentration of the particle-forming material in the suspension, the nature of the solvent and non-solvent, the rate at which the suspension is fed into the spray-drying apparatus and the temperature of the gas in the chamber. Usually, small size distributions can be achieved by a combination of a low suspension feed rate, a high degree of atomization and high flow rate of gas.

It is particularly preferred that, between step a) and step b), ie after formation of the suspension but before spray-drying, the suspension is subjected to homogenisation, eg by mechanical agitation. This results in a more even size distribution of particles in the suspension, and a correspondingly smaller and more even size distribution of the microparticles formed in step b).

The particle-forming material is most preferably proteinaceous, which includes non-naturally occurring polypeptides and polyamino acids. For example, the particle-forming material may be collagen, gelatin or albumin. Albumin is a particularly preferred material. Where the microparticles are intended for administration to the human body, the particle-forming material is preferably of human origin, ie actually derived from humans or identical (or substantially so) in structure to protein of human origin. A particularly preferred particle-forming material is thus human serum albumin which may, for instance, be obtained from donated blood or may be derived from the fermentation of microorganisms (including cell lines) that have been transformed or transfected to express human serum albumin. Non-proteinaceous materials that may be used as particle-forming materials include sugars, carbohydrates, drugs and materials useful as imaging contrast agents.

The suspension preferably contains from 0.1 to 50% w/v of particle-forming material, more preferably 1 to 20% w/v, and most preferably 2 to 10% w/v, particularly when the particle-forming material is albumin. Mixtures of particle-forming materials may be used, in which case the above figures represent the total content of particle-forming material(s).

The method according to the invention is advantageous primarily in that the two stages of the process (formation of a suspension and spray-drying) may be optimised separately to achieve the desired form and size distribution of microparticle. This gives a high degree of control over the properties of the microparticles. In particular, the process enables the production of microparticles with particularly small sizes and particularly narrow size distributions. Microparticles produced in accordance with the invention may, for example, have particle sizes of predominantly less than 4µm, and number sizes with modal peaks below 1µm and mean sizes (as measured using a Coulter counter) less than 2µm. Microparticles with such small sizes are beneficial in that they may enter very small blood vessels (capillaries) and/or may penetrate deep into the lungs. It may also be possible to produce microparticles of larger size, eg for nasal administration.

It will be appreciated that references to the "size" of the microparticles will normally mean the "diameter" of the microparticles, since the microparticles will most commonly be substantially spherical. However, it will also be appreciated that the microparticles may not be spherical, in which case the size may be interpreted as the diameter of a notional spherical particle having a mass equal to that of the non-spherical microparticle.

Furthermore, microparticles produced by spray-drying a suspension in accordance with the invention tend to be solid rather than the hollow microparticles that are typically produced when a solution is spray-dried.

In addition to the advantages noted above, the microparticles produced in accordance with the invention may be devoid of potentially undesirable excipients such as surfactants. Protein microparticles produced by spray-drying a suspension appear to suspend more readily in aqueous suspension without the need for surfactants in comparison with microparticles produced by other methods.

Microparticles produced by spray-drying a suspension are mis-shapen. This may change their flow properties and deaggregation properties compared to round/spherical microparticles that are produced by spray-drying a solution.

The precipitated particles produced in suspension can be coated with an agent to change their solubilisation properties. For example, cholesterol can be added to an iopamidol suspension in propan-2-ol/chloroform. The cholesterol will dissolve in the solvent mixture. On spray-drying the iopamidol suspension, the aqueous dissolution properties of the iopamidol microparticles will be changed. The cholesterol will coat the iopamidol microparticles, reducing their aqueous solubility.

If the solution contains two or more compounds that are not soluble in the "non-solvent", when the non-solvent is added a precipitate will form from both compounds. If the suspension is then spray-dried it will produce "mixed" microparticles containing both compounds.

The microparticles produced in accordance with the invention may be useful in therapeutic applications, eg vehicles for the delivery of medicaments, or in diagnostic imaging, eg for imaging techniques using ultrasound, magnetic resonance etc. The particle-forming material may therefore be a therapeutically active agent (ie a drug) or a pharmaceutical excipient, eg cholesterol, or it may be a contrast-enhancing agent for use in diagnostic imaging. Examples of contrast-enhancing agents are X-ray contrast agents, eg iopamidol, nuclear imaging agents, eg technetium, and magnetic resonance contrast agents. Therapeutically active agents may be incorporated into the microparticles, eg by absorption into and/or adsorption onto or covalent binding to the surface of the microparticles. Alternatively, the microparticles may be formed wholly or in part from the therapeutically active agent(s), ie the therapeutically active agent(s) may constitute, or be part of, the particle-forming material.

The invention will now be illustrated in greater detail, by way of illustration only, with reference to the following Examples and Figures, in which
Figure 1 is a light micrograph (x1000) showing morphology of microparticles produced when precipitated human albumin is spray-dried;
Figure 2 is a Coulter Size Distribution of albumin microparticles produced by spray-drying a suspension;
Figure 3 is a Coulter Size Distribution of albumin microparticles produced by spray-drying a solution;
Figure 4 is a light micrograph (x1000) showing morphology of microparticles produced when soluble human albumin is spray-dried;
Figure 5 is a light micrograph (x1000) showing morphology of microparticles produced when cholesterol solution is spray-dried;
Figure 6 is a light micrograph (x1000) showing morphology of microparticles produced when cholesterol suspension is spray-dried;
Figure 7 is a light micrograph (x1000) showing morphology of microparticles produced when iopamidol solution is spray-dried; and
Figure 8 is a light micrograph (x1000) showing morphology of microparticles produced when iopamidol suspension is spray-dried;

### Example 1

### Spray-drying of precipitated human albumin

### Method

A 100ml volume solution of Human Serum Albumin (20%w/v, USP grade) was dialysed against 2000ml volumes of pyrogen-free purified water (PFPW) overnight at room temperature to remove excess sodium chloride.

The following solution was prepared, with all work being done at 24°C, and the ethanol being added slowly, with gentle homogenisation with a Janke and Kunkel T25 homogeniser (8000rpm) whilst adding the ethanol:

| | |
|---|---|
| Dialysed Human Serum Albumin | 40ml |
| (12% w/v), pH 7.0 | |
| Ethanol | 60ml |

It was noted that the straw-coloured solution changed to a milky-white suspension when the ethanol was added.

The suspension was further homogenised using a Janke & Kunkel T25 homogeniser at 20,500 rpm for 30 seconds.

The homogenised suspension was spray-dried at the following settings using a Buchi Mini Spray Dryer model B-191, fitted with a Schlick 2-fluid atomisation nozzle (model 970/0). The following spray-drying conditions were used:

| | |
|---|---|
| Inlet temperature | 100°C |
| Starting outlet temperature | 67°C |
| Liquid feed rate | 3ml/min |
| Atomisation gauge pressure | 4.0 kPa (bar) |
| Drying air setting | 100% |

The microparticles were recovered from the cyclone collection jar.

To enable aqueous sizing on a Coulter counter, a 200mg sample of the microcapsules were rendered insoluble by heating in a Gallenkamp laboratory oven at 175°C for 60 minutes.

The insoluble microparticles were de-agglomerated (to break up aggregates) using a Fritsch Pulverisette 14 centrifugal mill running at maximum speed with a 12-tooth rotor and 0.5mm screen.

To achieve de-agglomeration the 200mg aliquot of heat fixed microcapsules was mixed with 600mg of anhydrous mannitol and blended with a spatula. The mixture was fed into the mill over a 10 second period whilst the mill was operating at maximum speed.

### Light Micrography

A small quantity (approx 100mg) of the deagglomerated microparticles were suspended in 5ml of water. Light microscopy was used to determine the particle morphology in the aqueous suspension. A small drop of suspension was placed on a microscope slide and a cover slip applied. An image at x1000 magnification was obtained using a Nikon Labophot microscope.

Images were captured using a SeeScan image analyser. The captured images were printed using a Sony Mavigraph colour video printer.

### Sizing

Prior to sizing, 50mg of the de-agglomerated microcapsules were re-suspended in 1ml of PFPW and vortexed gently. The microcapsules were sized in Isoton on a Coulter Multisizer II fitted with a 70µm orifice tube.

### Results

The de-agglomerated microcapsules exhibited excellent re-suspension properties in the PFPW. No surfactant addition was necessary to achieve good re-suspension.

The microparticles were found to have a small mean size (number distribution) of 1.97µm and the following size distribution (by number, measured as described above):

| % greater than | |
|---|---|
| 3µm | 7.51% |
| 4µm | 0.92% |
| 5µm | 0.13% |
| 6µm | 0.03% |

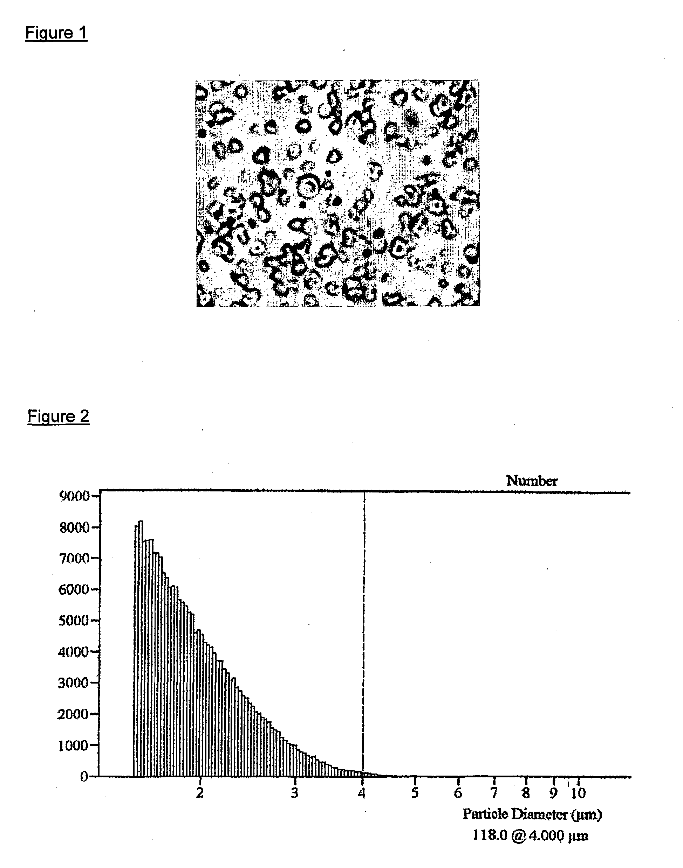

The images obtained by light micrography (see Figure 1) confirmed that the microparticles produced by spray-drying of precipitated albumin were somewhat mishapen. Closer analysis confirmed that the majority of the microparticles were not hollow in appearance.

The Coulter size distribution is shown in Figure 2.

### Example 2

### Spray-drying of human albumin as a solution (comparative Example)

### Method

In contrast to spray-drying a precipitated suspension (Example 1), human albumin was spray-dried as a solution.

A 250 ml volume of human albumin (USP Grade, supplied as 20% w/v by Grifols, Spain) was dialysed against 5.0 litres of purified water. The albumin was sealed in dialysis tubing and the water stirred overnight at room temperature.

The albumin concentration was determined by absorbance at 280 nm (assume an extinction coefficient of 0.53 for a 1.0mg/ml solution).
The following solution was prepared for spray-drying:
Human Albumin 4.8% (w/v)* in water, pH 7.0.
(* note that this is the same albumin concentration as in Example 1)

The albumin solution was spray-dried under the same conditions as in Example 1

The microparticles were collected and heat-insolubilised as described in Example 1.

For further analyses, the heat-insolublised microparticles were sonicated in ethanol to ensure that any agglomerates were disrupted. On subsequent aqueous suspension the microparticles were seen to be dispersed with no significant agglomeration (determined by light microscopy)

The microparticles were sized using a Coulter Multisizer as described above (Example 1)

The microparticles were subjected to light microscopy (x1000 magnification) as described in Example 1.

### Results

Coulter size analysis revealed that the microparticles had the following sizes:

| %greater than | |
|---|---|
| 3µm | 22.69% |
| 4µm | 7.15% |
| 5µm | 1.80% |
| 6µm | 0.39% |

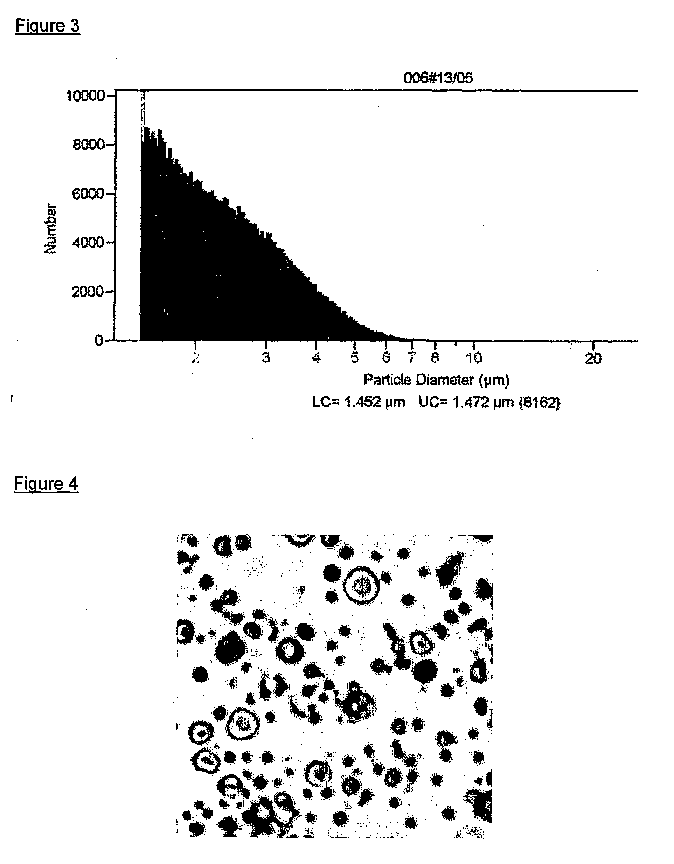

The size data (see Figure 3) confirmed that the microparticles produced by spray-drying an albumin solution were significantly larger than those produced by spray-drying a precipitated suspension of the same albumin concentration (Example 1).

Closer (light microscopic) analysis revealed that the microparticles produced by spray-drying an albumin solution were predominantly spherical in shape and predominantly hollow. Intact microparticles contained air and appeared as "black" bubbles under the microscope. In contrast the microparticles produced by spray-drying a suspension were somewhat mishapen. They were not hollow/air-containing.

A typical light micrograph is shown in Figure 4.

### Example 3

### Spray-drying of cholesterol as a suspension, compared to a solution

Cholesterol is a pharmaceutical excipient that can be used to formulate a variety of drugs. These applications include oral, topical and injectable drug formulations.

Cholesterol is insoluble in water but can be dissolved in organic solvents such as propan-2-ol and chloroform.

A comparative study was undertaken in which cholesterol was spray-dried as a solution (in propan-2-ol) compared to a suspension (precipitated from propan-2-ol by addition of a non-solvent, water).

### Method

A 2.5g quantity of cholesterol (Sigma, 99+%) was dissolved in 125 ml of propan-2-ol.

### Spray-drying as a solution:

A 50ml volume of the cholesterol solution was spray-dried using a Buchii spray dryer (see Example 1 for dryer specification).

The following spray-drying conditions were used:

| | |
|---|---|
| Inlet temperature | 50°C |
| Outlet temperature | 42°C |
| Liquid feed rate | 1.5ml/min |
| Atomisation gauge pressure | 1.0 kPa (bar) |
| Drying air setting | 100% |

The cholesterol solution was spray-dried to yield a fine white powder that was collected by the spray dryer cyclone.

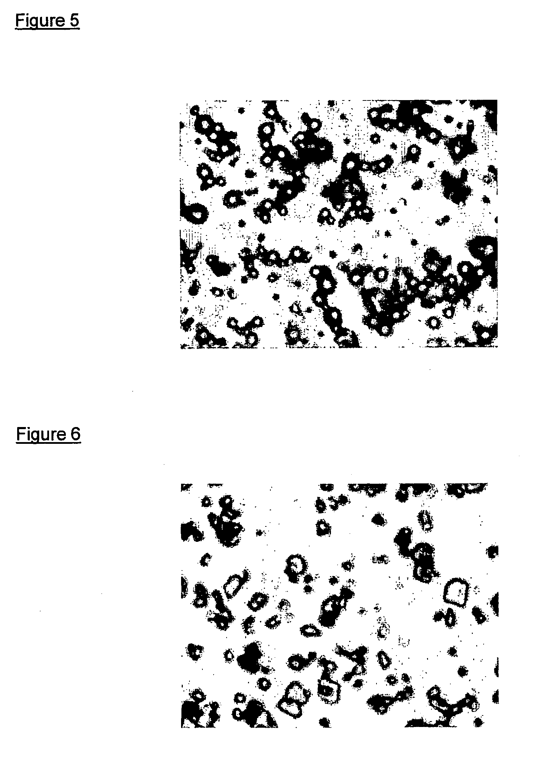

Light microscopy was undertaken on the dry powder collected. The microparticles were spread onto the surface of a microscope slide and analysed using a Nikon Labophot microscope (x1000 magnification) as described in Example 1 above. A typical light micrograph is shown in Figure 5.

### Spray-drying as a suspension:

The remaining 75 ml of cholesterol solution was placed on a magnetic stirrer and stirred at a medium setting.

The cholesterol was precipitated by addition of a non-solvent. In this example precipitation was achieved by slowly adding 25ml of purified water (over a period of approx 30 seconds).

The milky white suspension was spray-dried using the same conditions as described for the spray-drying of cholesterol solution. The microparticles produced were collected and analysed by light microscopy as described for the above sample. A typical light micrograph is shown in Figure 6.

### Results

The results obtained confirm that the cholesterol microparticles produced by spray-drying of a solution are predominantly spherical in appearance and appear to be hollow.

In contrast the cholesterol microparticles produced by spray-drying a suspension were mishappen and appeared crystalline in appearance.

### Example 4

### Spray-drying of iopamidol as a suspension, compared to a solution

Iopamidol is an X-ray contrast agent that is used primarily to enhance images of major blood vessels such as the coronary arteries. It is supplied as a sterile solution for injection. It is highly soluble in water.

There are several potential applications in which a dry powder formulation of the contrast agent may be advantageous. For example, dry microparticles of iopamidol can be further treated to produce an encapsulated formulation that offers the possibilty of targetting the contrast agent to other organs (such as the liver).

A comparative study was undertaken in which iopamidol was spray-dried as a solution (in propan-2-ol) compared to a suspension (precipitated from propan-2-ol by addition of chloroform).

### Method

### Spray-drying as a solution:

Commercially available iopamidol was diluted to give a solution for spray-drying:

| | |
|---|---|
| iopamidol* | 10ml |
| Water | 65ml |
| Ethanol | 25ml |

| | |
|---|---|
| (* Commercially available contrast agent with a concentration of 612mg iopamidol/ml) | |

The solution was spray-dried using a Buchii spray dryer (see Example 1 for dryer specification).

The following spray-drying conditions were used:

| | |
|---|---|
| Inlet temperature | 60°C |
| Outlet temperature | 47°C |
| Liquid feed rate | 1.5ml/min |
| Atomisation gauge pressure | 2.0 kPa (bar) |
| Drying air setting | 100% |

The iopamidol solution was spray-dried to yield a fine white powder that was collected by the spray dryer cyclone.

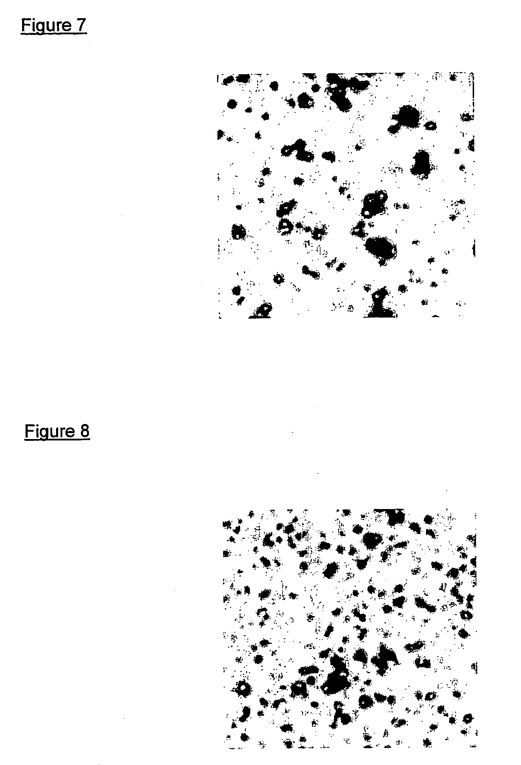

Light microscopy was undertaken on the dry powder collected. The microparticles were spread onto the surface of a microscope slide and analysed using a Nikon Labophot microscope (x1000 magnification) as described in Example 1 above. A typical light micrograph is shown in Figure 7.

### Spray-drying as a suspension:

Iopamidol was obtained as a dry powder for use in this study. The dry iopamidol was obtained by spray-drying a commercially available solution of iopamidol as described above.

A 2.0g quantity of dry iopamidol was dissolved in 50ml of propan-2-ol using a magnetic stirrer and also warming slightly to aid dissolution.

The iopamidol solution was precipitated by the addition of 50ml of chloroform (a miscible non-solvent) whilst stirring at medium speed. The chloroform was added over a 30 second period.

The suspension was spray-dried using a Buchii spray dryer (see Example 1 for dryer specification).

The following spray-drying conditions were used:

| | |
|---|---|
| Inlet temperature | 60°C |
| Outlet temperature | 47°C |
| Liquid feed rate | 1.5ml/min |
| Atomisation gauge pressure | 2.0 kPa (bar) |
| Drying air setting | 100% |

The iopamidol suspension was spray-dried to yield a fine white powder that was collected by the spray dryer cyclone.

Light microscopy was undertaken on the dry powder collected. The microparticles were spread onto the surface of a microscope slide and analysed using a Nikon Labophot microscope (x1000 magnification) as described in Example 1 above. A typical light micrograph is shown in Figure 8.

### Results

The results obtained confirm that the iopamidol microparticles produced by spray-drying of a solution are predominantly spherical in appearance and appear to be hollow (see Figure 7).

In contrast the iopamidol microparticles produced by spray-drying a suspension were mishappen and appeared crystalline in appearance. They also appeared somewhat smaller than the microparticles produced from a solution (see Figure 8).

## Claims

1. A method for producing microparticles of a particle-forming material, which method comprises the steps of
a) forming a suspension of the particle-forming material; and
b) spray-drying said suspension;
**characterised in that** step a) is carried out by first dissolving the particle-forming material in a solvent, and then adding to the solution so formed a non-solvent for the particle-forming material, so as to bring about precipitation of the particle-forming material.

2. A method as claimed in Claim 1, wherein the volume of non-solvent added to the solvent is greater than the volume of the solution of the particle-forming material in the solvent.

3. A method as claimed in Claim 2, wherein the solvent / non-solvent mixture that is spray-dried in step b) comprises in excess of 60% v/v of non-solvent.

4. A method as claimed in any preceding claim, wherein the solvent is water.

5. A method as claimed in any preceding claim, wherein the non-solvent is ethanol.

6. A method as claimed in any one of Claims 1 to 3, wherein the solvent is an organic solvent.

7. A method as claimed in any preceding claim, wherein spray-drying of the suspension formed in step a) is carried out by spraying the suspension into a chamber containing a heated gas.

8. A method as claimed in any preceding claim, wherein, between step a) and step b), the suspension is subjected to homogenisation.

9. A method as claimed in any preceding claim, wherein the particle-forming material is proteinaceous.

10. A method as claimed in Claim 9, wherein the proteinaceous material is albumin.

11. A method as claimed in Claim 10, wherein the albumin is human serum albumin.

12. A method as claimed in any one of Claims 9 to 11, wherein step a) is carried out by addition to a solution of the particle-forming material of a non-solvent for the particle-forming material, at a pH which is removed from the isoelectric point of the proteinaceous particle-forming material.

13. A method as claimed in any preceding claim, wherein the suspension contains from 0.1 to 50% w/v of particle-forming material.

14. A method as claimed in any preceding claim, wherein the suspension contains from 1 to 20% w/v of particle-forming material.

15. A method as claimed in any preceding claim, wherein the suspension contains from 2 to 10% w/v of particle-forming material.

16. A method as claimed in any preceding claim, wherein the particle-forming material is a therapeutically active agent.

17. A method as claimed in any one of Claims 1 to 15, wherein the particle-forming material is a pharmaceutical excipient.

18. A method as claimed in Claim 17, wherein the pharmaceutical excipient is cholesterol.

19. A method as claimed in any one of Claims 1 to 15, wherein the particle-forming material is an imaging contrast enhancing agent.

20. A method as claimed in Claim 19, wherein the contrast enhancing agent is an X-ray imaging contrast agent.

21. A method as claimed in Claim 20, wherein the contrast agent is iopamidol.

## Patentansprüche

1. Verfahren zur Herstellung von Mikropartikeln aus einem Partikel bildenden Material, wobei das Verfahren folgende Schritte umfasst:
a) Ausbilden einer Suspension des Partikel bildenden Materials und
b) Sprühtrocknen der Suspension,
**dadurch gekennzeichnet, dass** Schritt a) durchgeführt wird, indem zuerst das Partikel bildende Material in einem Lösungsmittel gelöst wird und dann der so ausgebildeten Lösung ein Nichtlöser für das Partikel bildende Material zugegeben wird, um die Ausfällung des Partikel bildenden Materials zu bewirken.

2. Verfahren nach Anspruch 1, wobei das Volumen des dem Lösungsmittel zugegebenen Nichtlösers größer ist als das Volumen der Lösung des Partikel bildenden Materials im Lösungsmittel.

3. Verfahren nach Anspruch 2, wobei das Lösungsmittel/Nichtlöser-Gemisch, das in Schritt b) sprühgetrocknet wird, mehr als 60 % (Vol./Vol.) Nichtlöser umfasst.

4. Verfahren nach einem vorangehenden Anspruch, wobei das Lösungsmittel Wasser ist.

5. Verfahren nach einem vorangehenden Anspruch, wobei der Nichtlöser Ethanol ist.

6. Verfahren nach einem der Ansprüche 1 bis 3, wobei das Lösungsmittel ein organisches Lösungsmittel ist.

7. Verfahren nach einem vorangehenden Anspruch, wobei das Sprühtrocknen der in Schritt a) ausgebildeten Suspension durchgeführt wird, indem die Suspension in eine ein erhitztes Gas enthaltende Kammer gesprüht wird.

8. Verfahren nach einem vorangehenden Anspruch, wobei die Suspension zwischen Schritt a) und Schritt b) homogenisiert wird.

9. Verfahren nach einem vorangehenden Anspruch, wobei das Partikel bildende Material proteinisch ist.

10. Verfahren nach Anspruch 9, wobei das Proteinmaterial Albumin ist.

11. Verfahren nach Anspruch 10, wobei das Albumin humanes Serumalbumin ist.

12. Verfahren nach einem der Anspruche 9 bis 11, wobei Schritt a) durchgeführt wird, indem einer Lösung des Partikel bildenden Materials bei einem pH-Wert, der vom isoelektrischen Punkt des Partikel bildenden Eiweißmaterials entfernt ist, ein Nichtlöser für das Partikel bildende Material zugegeben wird.

13. Verfahren nach einem vorangehenden Anspruch, wobei die Suspension 0,1 bis 50 % (Gew./Vol.) Partikel bildendes Material enthält.

14. Verfahren nach einem vorangehenden Anspruch, wobei die Suspension 1 bis 20 % (Gew./Vol.) Partikel bildendes Material enthält.

15. Verfahren nach einem vorangehenden Anspruch, wobei die Suspension 2 bis 10 % (Gew.Nol.) Partikel bildendes Material enthält.

16. Verfahren nach einem vorangehenden Anspruch, wobei das Partikel bildende Material ein Therapeutikum ist.

17. Verfahren nach einem der Ansprüche 1 bis 15, wobei das Partikel bildende Material ein pharmazeutisches Bindemittel ist.

18. Verfahren nach Anspruch 17, wobei das pharmazeutische Bindemittel Cholesterol ist.

19. Verfahren nach einem der Ansprüche 1 bis 15, wobei das Partikel bildende Material ein Bildkontrastverstärkungsmittel ist.

20. Verfahren nach Anspruch 19, wobei das Kontrastverstärkungsmittel ein Röntgenbildkontrastmittel ist.

21. Verfahren nach Anspruch 20, wobei das Kontrastmittel lopamidol ist.

## Revendications

1. Procédé pour la production de microparticules d'un matériau formant des particules, lequel procédé comprend les étapes
a) de formation d'une suspension du matériau formant des particules ; et
b) de séchage par pulvérisation de ladite suspension ;
**caractérisé en ce que** l'étape a) est réalisée en dissolvant dans un premier temps le matériau formant des particules dans un solvant et en ajoutant ensuite à la solution ainsi formée un non-solvant pour le matériau formant des particules afin d'occasionner la précipitation du matériau formant des particules.

2. Procédé selon la revendication 1, dans lequel le volume de non-solvant ajouté au solvant est supérieur au volume de la solution du matérlau formant des particules dans le solvant.

3. Procédé selon la revendication 2, dans lequel le mélange solvant/non-solvant qui est séché par pulvérisation dans l'étape b) comprend un excès de 60 % volume/volume de non-solvant.

4. Procédé selon l'une quelconque des revendications précédentes, dans lequel le solvant est l'eau.

5. Procédé selon l'une quelconque des revendications précédentes, dans lequel le non-solvant est l'éthanol.

6. Procédé selon l'une quelconque des revendications 1 à 3, dans lequel le solvant est un solvant organique.

7. Procédé selon l'une quelconque des revendications précédentes, dans lequel le séchage par pulvérisation de la suspension formée dans l'étape a) est réalisé en pulvérisant la suspension dans une chambre contenant un gaz chauffé.

8. Procédé selon l'une quelconque des revendications précédentes, dans lequel la suspension est soumise à une homogénéisation entre l'étape a) et l'étape b).

9. Procédé selon l'une quelconque des revendications précédentes, dans lequel le matériau formant des particules est protéiné.

10. Procédé selon la revendication 9, dans lequel le matériau protéiné est l'albumine.

11. Procédé selon la revendication 10, dans lequel l'albumine est de la sérum-albumine humaine.

12. Procédé selon l'une quelconque des revendications 9 à 11, dans lequel l'étape a) est réalisée par addition à une solution du matériau formant des particules d'un non-solvant pour le matériau formant des particules, à un pH qui est redéplacé à partir du point iso-électrique du matériau formant des particules protéiné.

13. Procédé selon l'une quelconque des revendications précédentes, dans lequel la suspension contient de 0,1 à 50 % en poids/volume de matériau formant des particules.

14. Procédé selon l'une quelconque des revendications précédentes, dans lequel la suspension contient de 1 à 20 % en poids/volume de matériau formant des particules.

15. Procédé selon l'une quelconque des revendications précédentes, dans lequel la suspension contient de 2 à 10 % en poids/volume de matériau formant des particules.

16. Procédé selon l'une quelconque des revendications précédentes, dans lequel le matériau formant des particules est un agent thérapeutiquement actif.

17. Procédé selon l'une quelconque des revendications 1 à 15, dans lequel le matériau formant des particules est un excipient pharmaceutique.

18. Procédé selon la revendication 17, dans lequel l'excipient pharmaceutique est le cholestérol.

19. Procédé selon l'une quelconque des revendications 1 à 15, dans lequel le matériau formant des particules est un agent accentuant le contraste de la formation d'une Image.

20. Procédé selon la revendication 19, dans lequel l'agent accentuant le contraste est un agent de contraste pour la formation d'une image aux rayons X.

21. Procédé selon la revendication 20, dans lequel l'agent de contraste est l'iopamidol.
